# EUROPEAN PATENT APPLICATION

(11) **EP 4 625 425 A1**
(43) Date of publication of application: **01.10.2025**
(21) Application number: 24166605.6
(22) Date of filing: 27.03.2024
(51) Int. Cl.: G16H 10/40, G16H 40/60, G01N 35/04

(54) **A COMPUTER-IMPLEMENTED METHOD FOR MONITORING AND DOCUMENTING PROCESS STEPS IN A LABORATORY AND A RESPECTIVE DEVICE**

(71) Applicant: Nordmark Pharma GmbH, 25436 Uetersen (DE)
(72) Inventor: Meineke, Bernhard, 25436 Uetersen (DE)
(74) Representative: RGTH

(57) **Abstract**

The present invention relates to a computer-implemented method (100) for monitoring and documenting process steps in a laboratory, which comprises monitoring (113) a position of at least one object within the laboratory and documenting (121) the position of the object.

## Description

The present invention relates to a computer-implemented method as well as a device for monitoring and documenting process steps in a laboratory pursuant to the independent claims.

### Prior art

Proper documentation of experiments or any process steps conducted in a laboratory setting is challenging.

A potential problem is that the time of the documentation has a delay regarding the actual time of conducting the process steps. Usually, certain steps must be concluded first, before the documentation, i.e., the protocol, can be written and the next step can begin. This can result in multiple problematic issues. First, the process or in other words the experiment must be interrupted every time a process step needs to be documented. This can lead to mistakes when resuming the process. Further, since the documentation is delayed, there can be mistakes in the documentation itself. In addition, especially when guaranteeing good manufacturing practice (GMP), the documentation can be very time consuming.

Known from prior art are further fully automated systems to supervise or conduct experiments such as e.g., known from DE 10 2012 024 208 A1 or EP 0 990 909 A1. These fully automated laboratory systems which implement laboratory steps require an exact predefinition of the steps to be conducted and are thus very inflexible.

### Objective, solution, advantages

The present invention has the objective to provide a method for monitoring process steps in a laboratory which allows a reliable documentation of process steps but which at the same time is adaptive and flexible towards different processes.

The objective is solved by a method for monitoring process steps in a laboratory wherein the method is computer implemented and comprises monitoring a position of at least one object within the laboratory and documenting the position of the object. The term "process step" refers to an experimental step or laboratory step.

The monitoring can be based on a detected position of the at least one object by a sensor. Preferably, the sensor is configured as a camera which is adapted to recognize and then monitor a position of at least one object. The object can be detected by recognizing the shape of the object and a respective classification of the object. Further, a LIDAR or RADAR sensor can be used which can detect the shape of the object and its position within the room, especially its distance to the sensor. Especially, the position is monitored on a regular basis, especially permanently to detect every movement of the object. Thus, the monitoring is enabled by an object detection.

The method comprises documenting the position of the object which is preferably automatic. Thus, if it is recognized that the position has changed, this change in position is documented automatically. Preferably, the documentation is done digitally. In other words, the position and thus also change of position is stored.

The at least one object can be a laboratory tool, such as e.g., a pipette, or a pipette tip, and/or a vial and/or a container of a substance. A vial can be understood as a sample vessel. Especially, every laboratory tool, e.g., each pipette and/or vial and/or container of substances, present in the laboratory or regarding the process steps or an experiment is monitored regarding its respective position. The latter case relates to a prior definition and labelling of each laboratory tool necessary for the process steps or in other words the experiment to be conducted. The substance can be a fluid, especially a solution, which is for example used in combination with a pipette and/or a vial. In particular, the tip of the pipette, which can be disposable, can be monitored regarding its position.

Advantageously, the method comprises a prior labeling of the at least on object. This preferably relates to attaching a label to the object. Such a label can then be detected to monitor the position of the labelled object. Especially, each laboratory tool, e.g., each pipette and/or vial and/or container, present in the laboratory or regarding an experiment are labelled accordingly. Regarding pipettes, the respective tip can be labeled.

A prior labelling of the at least one object guarantees a particularly reliable detection without e.g., object classification. Furthermore, it allows an association of further data to the object. For example, for each container, the label can comprise information of the respective substance present in the container which can then be identified by recognizing the label. Especially, the label can be a QR-code or an RFID (radio-frequency identification) transponder which can easily be detected by the sensor. In addition, the label can be any kind of visual code or visual fiducial system, such as e.g., an AprilTag.

Alternative to attaching a label, an object can be monitored by object detection as explained above.

The method can comprise detecting at least one process step which involves the at least one object and documenting the process step. Involving means that the process step is conducted with at least one monitored object. For example, a substance can be taken up from a container by means of a pipette to a vial, wherein the container, the pipette and/or the vial can be a monitored object.

The method can comprise a prior definition of process steps, such that the method can automatically recognize process steps without further indication from a person or a robot conducting the process step. The prior definition can include which object is involved. Further, a person or a robot conducting the process step, can indicate, for example, by an electronic signal that a process step has been concluded. Such a signal can e.g., be triggered by an electronic push button. Furthermore, the electronic signal can be triggered by a sensor, such as e.g., a light sensor or a motion sensor. Such a sensor can comprise a neural network. The conveyed information by the electronic signal can include the type of the process step as well as further information, such as e.g., time, a volume of a substance involved in a process step and/or the objects involved.

Each process step, which is detected, can be documented automatically, preferably digitally. Documenting the process steps comprises documenting at least which objects have been involved. For example, a process step can be taking up or releasing a substance with a pipette. In this respect, it is important which substance has been taken up or released which can be easily detected by monitoring the position of the respective container which comprises the respective substance. A prior label can include information which substance is present in the container.

Thus, it can be documented which substance has been taken up and/or released with the pipette.

Especially, a process step can be detected by an electronic signal, which can e.g., be triggered by an electronic push button or a sensor. Furthermore, whether a substance is taken up or released can for example be detected by an electronic signal triggered by an electronic push button or a sensor. Such a push button or a sensor can be in direct functional relationship with the pipette in such a way that the push button must be pushed for the pipette to take up or release a respective substance or the sensor automatically triggers a respective signal if the pipette takes up or releases a respective substance. The push button can be placed directly on the respective pipette as a control button of the pipette. The same can apply to the sensor.

To detect how much volume of a substance has been taken up or released can also be detected by a respective push button or a sensor. For example, the time span during which the push button is pushed or a sensor is triggered can directly relate to a volume which is taken up or released by the pipette. Further, the pipette can comprise multiple push buttons or sensors which can relate to different process steps and/or volumes. In addition, the speed or the mode, e.g., the mode "reverse", of a pipetting process step can be determined and conveyed as an information by an electronic signal.

The detected volume can further be automatically documented as part of the process. Especially, a process step can include which substance and which volume has been placed with which another object, such as e.g., a pipette, in which other object, such as e.g., a vial.

Each detected step can be displayed on a display, especially directly after it has been recognized. Each involved object can be displayed along with the respective action such as e.g., taking up or releasing a substance. For example, the display could show which substance and which volume has been placed with which other object, such as e.g., a pipette, in which other object, such as e.g., a vial. If for example, a process has not been recognized correctly, a supervising person or a person conducting the process step, can intervene and for example delete, amend or repeat the documented step. The display can e.g., be a screen which can be placed in the laboratory. Further, the display can be a surface on which a projection is shown, e.g., by means of a laser galvanometer. The surface can be a work surface or a wall.

The present invention allows a real time monitoring of objects in a laboratory such that the process steps can be recognized and documented automatically. This results in a reliable, time efficient documentation process which is far less prone to errors resulting from manual, delayed documentation. Further, any process can be easily documented in a flexible way.

In particular, the method uses a neural network for monitoring and/or documenting the position of at least one object and/or for detecting and/or documenting the at least one process step.

In particular, the method can comprise creating a step-by-step documentation of a monitored process. By monitoring a position of at least one object and documenting the position, process steps can be recognized and documented. Thus, a step by step documentation of a complete process can be created in an easy and time-efficient way. In a way, that step by step documentation can be used as a manual for repeating the monitored process to produce reliable results and/or to find discrepancies in the process steps in an efficient way if results deviate.

The method can comprise supervising a repetition of a monitored process step conducted by a person or a robot. Thus, the method can include a first mode in which a process is monitored, and preferably a step-by-step documentation is created, and a second mode in which a repetition of the monitored process can be supervised. For example, a person or a robot who or which is to conduct the process can be instructed to conduct each step of the documented process. Thus, the step-by-step documentation can be used as a manual to supervise the repetition.

Each step which is to be conducted can be shown on a display which can, as explained above, be a work surface. For example, each involved object can be displayed along with the action to be conducted. Furthermore, the volume of the substance to be taken up or released can be shown.

The method can comprise recognizing differences in a conducted process step with regard to a documented process step and documenting these differences. Thus, while the method can comprise instructing the person and/or the robot, it can monitor the position of the objects during the repetition and recognize the actual steps which are conducted. These actual steps can be compared with the step to be conducted according to the documentation. If there are differences, these can automatically be documented. The method can also comprise issuing a warning signal if a step is not conducted according to the documentation. Deviations from the documentation can be expected differences, e.g., that a pipetting process has not yet started, or a positioning is not yet correct. These differences to the documentation can be issued, for example by means of the display. For example, steps to be conducted which have not been initiated yet, could be displayed in a different colour than steps which have been concluded.

Overall, the supervision allows for the person or for the robot to conduct the step to intervene, such that, if possible, the process step can be corrected, or the process step or process must be repeated.

In another aspect of the invention, it relates to a device for monitoring a process in a laboratory which comprises an analysis module for monitoring a position of at least one object within the laboratory and documenting its position. The analysis module is preferably configured to detect and document at least one process step. Especially, the device is configured to conduct an above-described method.

The device can further comprise a display for displaying recognized process steps and/or for displaying steps to be conducted in a repetition mode. Further, the device can comprise a storing module for documenting, in other words storing the process steps and/or the position of the objects.

The analysis module can act as a control unit controlling the display as well as the storing module. It can also decide whether a warning signal is to be issued, for example by means of the display or by a tone or an audio warning.

The device can further comprise a detection module. The detection module can include a sensor for monitoring a position of at least one object, e.g., a camera, and can comprise at least one push button or a sensor, for example of a pipette, which can indicate relevant information of process steps which have been conducted. Such information can be a volume of a substance involved in a process step and/or the type of the process step and/or the objects involved.

### Brief description of the figures

Only schematically, the figures show:
- Figure 1:: a flow diagram of a computer implemented method for monitoring process steps in a laboratory; and
- Figure 2:: a device for monitoring process steps in a laboratory.

### Preferred embodiments

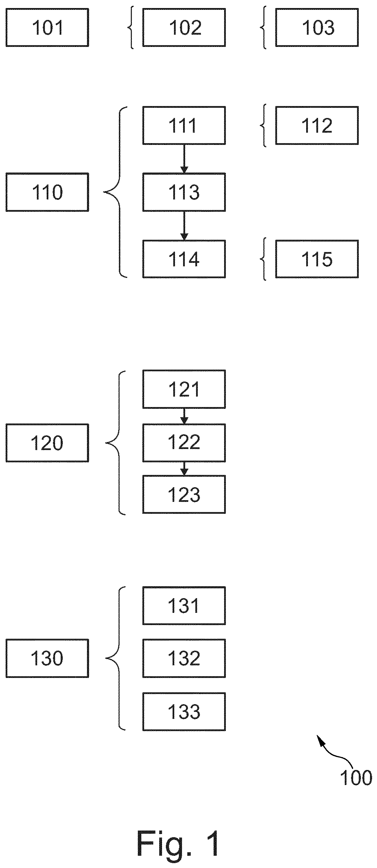

In figure 1 a computer implemented method 100 for monitoring process steps in a laboratory is shown.

The method 100 can comprise a preparation 101 which might include labelling 102 at least one object, for example attaching 103 a QR-code. Furthermore, the method 100 includes monitoring 113 a position of at least one object by at least one camera and documenting 121 the position. The monitoring 113 can be part of monitoring 110 a process. Especially, the position of the object is detected 111 by a sensor. Preferably, the label is detected 112. Furthermore, at least one process step involving at least one object is detected 114 which can for example be achieved by detecting 115 an electronic signal triggered by an electronic push button or a sensor. Documenting 121 the position of the at least one object can be part of documenting 120 the monitored process. Especially, the method 100 comprises documenting 122 the at least one process step which has been recognized. Furthermore, a step-by-step documentation of the monitored process can be created 123.

All these steps can relate to a first mode of the method which concerns the monitoring. In a second mode, a repetition of the process can be supervised 130. For example, it can be indicated 132 that each step is conducted according to the documentation and/or a warning signal can be issued 133, if the step is not conducted according to the documentation. Furthermore, a person or a robot to conduct the step each step can be instructed 131.

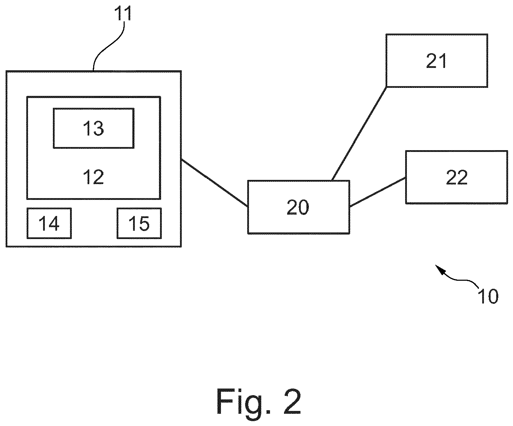

Figure 2 shows a device 10 for monitoring process steps in a laboratory which can have a detection module 11 including a sensor, especially a camera 13, to detect the position of at least one object. Especially, prior attached labels can be detected and thus the position of the respective object can be monitored.

Furthermore, the detection module 11 can include push buttons or sensors which can trigger an electronic signal indicating information on a conducted process step. A push button or a sensor 14 can be implemented in the laboratory or a push button or a sensor 15 can be specifically assigned to an object, such as a pipette. Furthermore, the device 10 can include an analysis module 20 which is configured to monitor the position of the at least object. The analysis module 20 can be configured to recognize and document process steps and thus to monitor an entire process. It can further be configured to create a step-by-step documentation of a process.

The device 10 can further include a display 21 to display process steps which are recognized or process steps to be conducted in a repetition mode. Furthermore, the device 10 can have a storing module 22 for documenting the position of objects and/or recognized steps and/or storing the step-by-step documentation.

### Reference signs

- 100: method
- 101: preparation
- 102: labelling at least one object
- 103: attaching a QR-code or an RFID transponder

- 110: monitoring a process
- 111: detecting a position by a sensor
- 112: detecting a label on at least one object
- 113: monitoring a position of at least one object by at least one camera
- 114: detecting at least one process step involving at least one object
- 115: detecting an electronic signal triggered by an electronic push button or a sensor

- 120: documenting the process
- 121: documenting the position of the at least one object
- 122: documenting the at least one process step
- 123: creating a step by step documentation of the monitored process

- 130: supervising a repetition of the process conducted by a person or a robot
- 131: instructing the person or the robot to conduct each step of the documented process
- 132: indicating if each step is conducted according to the documentation
- 133: issuing a warning signal if a step is not conducted according to the documentation

- 10: device
- 11: detection module
- 12: sensor
- 13: camera
- 14: push button or sensor implemented in laboratory
- 15: push button or sensor assigned to an object
- 20: analysis module
- 21: display
- 22: storing module

## Claims

1. A computer-implemented method (100) for monitoring and documenting process steps in a laboratory,
**characterized in that**
the method (100) comprises monitoring (113) a position of at least one object within the laboratory and documenting (121) the position of the object.

2. The method (100) according to claim 1,
**characterized in that**
the monitoring (113) is based on a detected position of the at least one object by a sensor.

3. The method (100) according to claim 1 or 2,
**characterized in that**
the at least one object is a laboratory tool, such as a pipette and/or a vial and/or a container of at least a substance.

4. The method (100) according to any one of the preceding claims,
**characterized in that**
the method (100) comprises a prior labelling (102) of the at least one object and detecting (112) the label to monitor the position of the labelled object.

5. The method (100) according to claim 4,
**characterized in that**
the label is a QR-code or an RFID transponder.

6. The method (100) according to any one of the preceding claims,
**characterized in that**
the method (100) comprises detecting (114) at least one process step involving the at least one object and documenting (122) the process step.

7. The method (100) according to claim 6,
**characterized in that**
the process step is taking up and/or releasing of substance with a pipette.

8. The method (100) according to claim 6 or 7,
**characterized in that**
the process step is detected (114) by detecting (115) an electronic signal triggered by an electronic push button or a sensor.

9. The method (100) according to any one of the preceding claims,
**characterized in that**
the method (100) uses a neural network for monitoring (113) and/or documenting (121) the position of the at least one object and/or for detecting (114) and/or documenting (122) the at least one process step.

10. The method (100) according to any one of claims 6 to 9,
**characterized in that**
the method (100) comprises creating (123) a step-by-step documentation of a monitored process.

11. The method (100) according to any one of the preceding claims,
**characterized in that**
the method (100) comprises supervising (130) a repetition of a monitored process conducted by a person or a robot.

12. The method (100) according to claim 11,
**characterized in that**
the method (100) comprises instructing (131) the person or the robot to conduct each step of the documented process.

13. The method (100) according to claim 11 or 12,
**characterized in that**
the method (100) comprises recognizing differences in a conducted process step with regard to a documented process step.

14. The method (100) according to any one of claims 11 to 13,
**characterized in that**
the method (100) comprises issuing (132) a warning signal if a step is not conducted according to the documentation.

15. A device (10) for monitoring and documenting process steps in a laboratory,
**characterized in that**
the device (10) comprises an analysis module (20) for monitoring a position of at least one object within the laboratory and documenting the position of the object.
